# EUROPÄISCHE PATENTSCHRIFT

(11) **EP 1 345 939 B1**
(45) Veröffentlichungstag und Bekanntmachung des Hinweises auf die Patenterteilung: **07.06.2006**
(21) Anmeldenummer: 01271374.9
(22) Anmeldetag: 05.12.2001
(51) Int. Cl.: C07D 487/04, C07D 253/00, C07D 235/00

(54) **VERFAHREN ZUR HERSTELLUNG VON SULFONAMID-SUBSTITUIERTEN IMIDAZOTRIAZINONEN**
METHOD FOR PRODUCING SULFONAMIDE-SUBSTITUTED IMIDAZOTRIAZINONES
PROCEDE DE PRODUCTION D'IMIDAZOTRIAZINONES A SUBSTITUTION SULFONAMIDE

(30) Priorität: 18.12.2000 DE 10063108
(43) Veröffentlichungstag der Anmeldung: 24.09.2003
(73) Patentinhaber: Bayer HealthCare AG, 51368 Leverkusen (DE)
(72) Erfinder: NOWAKOWSKI, Marc, 42115 Wuppertal (DE); GEHRING, Reinhold, 42327 Wuppertal (DE); HEILMANN, Werner, 42327 Wuppertal (DE); WAHL, Karl-Heinz, 51519 Odenthal (DE)
(86) Internationale Anmeldenummer: PCT/EP2001/014239
(87) Internationale Veröffentlichungsnummer: WO 2002/050076

(56) Entgegenhaltungen:
- WO-A-99/24433

## Beschreibung

Die vorliegende Erfindung betrifft ein Verfahren zur Herstellung von Sulfonamid-substituierten Imidazotriazinonen.

Es ist bekannt, dass Verbindungen, welche in der Lage sind, cyclisches Guanosin-3',5'-monophosphat metabolisierende Phosphodiesterasen (cGMP-PDE's) zu inhibieren, zur Behandlung von Impotenz eingesetzt werden können (vgl. z.B. EP-B-0 702 555; K. Murray, Drugs, News & Perspectives 6 (1993), 150).

In der WO 99/24433 sind Sulfonamid-substituierte Imidazotriazinone als potente Inhibitoren von entweder einer oder mehrerer der cyclisches Guanosin-3',5'-monophosphat metabolisierenden Phosphodiesterasen (cGMP-PDE's) beschrieben. Entsprechend der Nomenklatur von Beavo und Reifsnyder (Trends in Pharmacol. Sci. 11, 150-155, 1990) handelt es sich bei diesen cGMP-PDE's um die Phosphodiesterase-Isoenzyme PDE-I, PDE-II und PDE-V.

Gemäß der WO 99/24433 werden die dort beschriebenen Sulfonamid-substituierten Imidazotriazinone aus entsprechenden 2-Ethoxyphenyl-substituierten Imidazotriazinonen durch Umsetzung mit Chlorsulfonsäure und anschließende Reaktion mit einem entsprechenden Amin hergestellt, wie durch folgendes Schema veranschaulicht wird (R¹ bis R⁶ haben hierbei die in der WO 99/24433 angegebenen Bedeutungen):

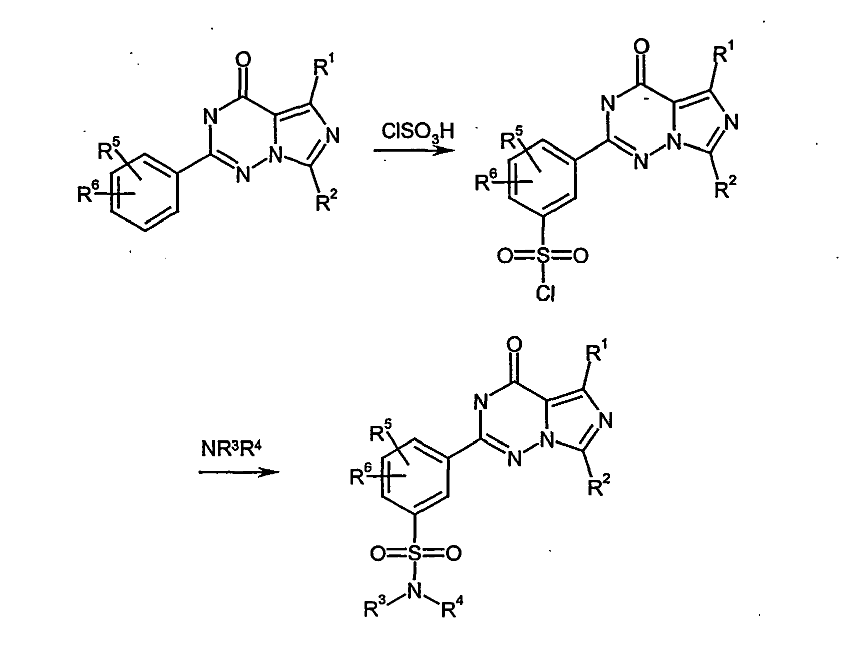

Bei diesem Verfahren muss hochreaktive Chlorsulfonsäure als Reagenz verwendet werden. Zudem sind die als Zwischenstufe anfallenden Imidazotriazinonsulfonylchloride hydrolyseempfindlich, was insbesondere bei der Umsetzung dieses Herstellungsverfahrens im großtechnischen Maßstab zu nicht unerheblichen Ausbeuteschwankungen führen kann.

Es war daher die Aufgabe der vorliegenden Erfindung, ein Verfahren zur Herstellung von Sulfonamid-substituierten Imidazotriazinonen bereitzustellen, bei welchem die Nachteile des aus dem Stand der Technik bekannten vorstehenden Verfahrens vermieden werden.

Diese Aufgabe wird gemäß der vorliegenden Erfindung durch ein Verfahren gemäß Anspruch 1 gelöst. Insbesondere wird bei dem erfindungsgemäßen Verfahren gemäß Anspruch 1 der Einsatz von Chlorsulfonsäure durch Einführung der Sulfonsäure über eine Reaktion mit Schwefelsäure und anschließende Umsetzung mit Thionylchlorid vermieden. Zudem wird die Reaktion mit Thionylchlorid und die anschließende Umsetzung mit einem Amin in einem Eintopfverfahren durchgeführt, so dass die hydrolyseempfindliche Imidazotriazinonsulfonylchlorid-Zwischenstufe nicht isoliert zu werden braucht. Dadurch können Ausbeuteschwankungen aufgrund partieller Hydrolyse dieser Zwischenstufe ausgeschaltet werden. Durch diese Vorteile ist das erfindungsgemäße Verfahren viel einfacher in den großtechnischen Maßstab umzusetzen als das in der WO 99/24433 beschriebene Verfahren.

Das erfindungsgemäße Verfahren umfasst die Herstellung von Verbindungen der Formel (I) 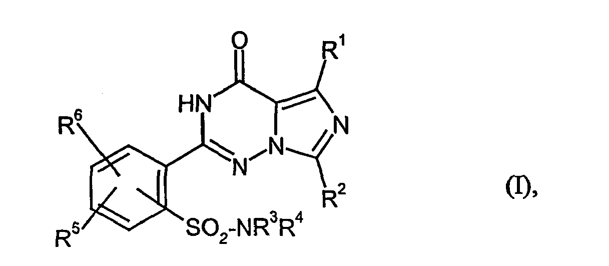 in welcher
- R¹: für Wasserstoff oder geradkettiges oder verzweigtes Alkyl mit bis zu 4 Kohlenstoffatomen steht,
- R²: für geradkettiges Alkyl mit bis zu 4 Kohlenstoffatomen steht,
- R³ und R⁴: gleich oder verschieden sind und für eine geradkettige oder verzweigte Alkylkette mit bis zu 5 Kohlenstoffatomen stehen, die gegebenenfalls bis zu zweifach gleich oder verschieden durch Hydroxy oder Methoxy substituiert ist,
oder
- R³ und R⁴: gemeinsam mit dem Stickstoffatom einen Piperidinyl-, Morpholinyl-, Thiomorpholinylring oder einen Rest der Formel 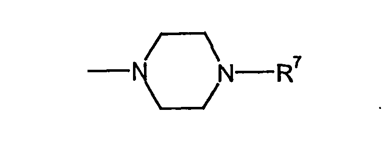
bilden,
worin
- R⁷: Wasserstoff, Formyl, geradkettiges oder verzweigtes Acyl oder Alkoxycarbonyl mit jeweils bis zu 6 Kohlenstoffatomen bedeutet, oder geradkettiges oder verzweigtes Alkyl mit bis zu 6 Kohlenstoffatomen bedeutet, das gegebenenfalls ein- bis zweifach, gleich oder verschieden durch Hydroxy, Carboxyl, geradkettiges oder verzweigtes Alkoxy oder Alkoxycarbonyl mit jeweils bis zu 6 Kohlenstoffatomen substituiert ist, oder C₃₋₈-Cycloalkyl bedeutet,
und die unter R³ und R⁴ aufgeführten, gemeinsam mit dem Stickstoffatom gebildeten Heterocyclen, gegebenenfalls ein- bis zweifach, gleich oder verschieden, gegebenenfalls auch geminal, durch Hydroxy, Formyl, Carboxyl, geradkettiges oder verzweigtes Acyl oder Alkoxycarbonyl mit bis jeweils zu 6 Kohlenstoffatomen substituiert sind,
und/oder die unter R³ und R⁴ aufgeführten, gemeinsam mit dem Stickstoffatom gebildeten Heterocyclen, gegebenenfalls durch geradkettiges oder verzweigtes Alkyl mit bis zu 6 Kohlenstoffatomen substituiert sind, das gegebenenfalls ein- bis zweifach, gleich oder verschieden durch Hydroxy, Carboxyl substituiert ist,
und/oder die unter R³ und R⁴ aufgeführten, gemeinsam mit dem Stickstoffatom gebildeten Heterocyclen, gegebenenfalls durch über N-verknüpftes Piperidinyl oder Pyrrolidinyl substituiert sind,
- R⁵ und R⁶: gleich oder verschieden sind und für Wasserstoff, geradkettiges oder verzweigtes Alkyl mit bis zu 6 Kohlenstoffatomen, Hydroxy oder für geradkettiges oder verzweigtes Alkoxy mit bis zu 6 Kohlenstoffatomen stehen,
dadurch gekennzeichnet, dass Verbindungen der Formel (II) 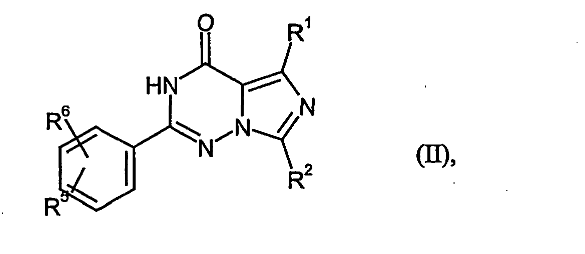 worin
R¹, R², R⁵ und R⁶ die vorstehend angegebenen Bedeutungen haben,
mit Schwefelsäure zu Verbindungen der Formel (III) 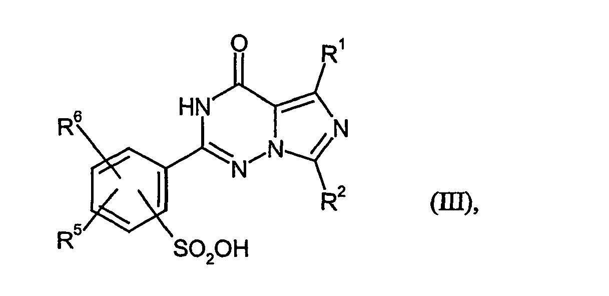 worin
R¹, R², R⁵ und R⁶ die vorstehend angegebenen Bedeutungen haben,
und anschließend mit Thionylchlorid und das so erhaltene Produkt in situ in einem inerten Lösungsmittel mit einem Amin der Formel (IV) 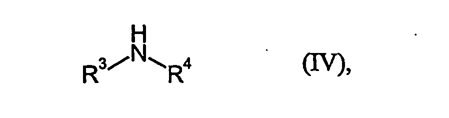 worin
R³ und R⁴ die vorstehend angegebene Bedeutung haben,
sowie gegebenenfalls zu den entsprechenden Salzen, Hydraten oder N-Oxiden
umgesetzt werden.

Gemäß einer bevorzugten Ausführungsform der vorliegenden Erfindung bedeuten bei den Reaktanden und dem Endprodukt des erfindungsgemäßen Verfahrens
- R¹: Wasserstoff oder geradkettiges oder verzweigtes Alkyl mit bis zu 4 Kohlenstoffatomen,
- R²: geradkettiges Alkyl mit bis zu 4 Kohlenstoffatomen,
- R³ und R⁴: gleich oder verschieden voneinander eine geradkettige oder verzweigte Alkylkette mit bis zu 5 Kohlenstoffatomen, die gegebenenfalls bis zu zweifach gleich oder verschieden durch Hydroxy oder Methoxy substituiert ist,
oder
- R³ und R⁴: gemeinsam mit dem Stickstoffatom einen Piperidinyl-, Morpholinylring oder einen Rest der Formel 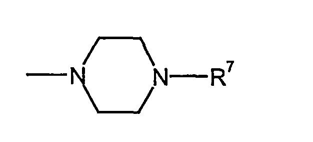
bilden,
worin
- R⁷: Wasserstoff, geradkettiges oder verzweigtes Alkyl mit bis zu 4 Kohlenstoffatomen bedeutet, das gegebenenfalls ein- bis zweifach, gleich oder verschieden durch Hydroxy, geradkettiges oder verzweigtes Alkoxy mit jeweils bis zu 4 Kohlenstoffatomen substituiert ist, oder C₃₋₆-Cycloalkyl bedeutet,
und die unter R³ und R⁴ aufgeführten, gemeinsam mit dem Stickstoffatom gebildeten Heterocyclen, sind gegebenenfalls ein- bis zweifach, gleich oder verschieden, gegebenenfalls auch geminal, durch Hydroxy, geradkettiges oder verzweigtes Acyl oder Alkoxycarbonyl mit bis jeweils zu 4 Kohlenstoffatomen, gegebenenfalls durch geradkettiges oder verzweigtes Alkyl mit bis zu 4 Kohlenstoffatomen substituiert sind, das gegebenenfalls ein- bis zweifach, gleich oder verschieden durch Hydroxy substituiert ist,
- R⁵ und R⁶: gleich oder verschieden voneinander Wasserstoff, geradkettiges oder verzweigtes Alkyl mit bis zu 6 Kohlenstoffatomen, Hydroxy oder für geradkettiges oder verzweigtes Alkoxy mit bis zu 6 Kohlenstoffatomen.

Gemäß einer besonders bevorzugten Ausführungsform der vorliegenden Erfindung bedeuten bei den Reaktanden und dem Endprodukt des erfindungsgemäßen Verfahrens
- R¹: Wasserstoff oder geradkettiges oder verzweigtes Alkyl mit bis zu 4 Kohlenstoffatomen,
- R²: geradkettiges Alkyl mit bis zu 4 Kohlenstoffatomen,
- R³ und R⁴: gleich oder verschieden voneinander Methyl oder Ethyl, die gegebenenfalls bis zu zweifach gleich oder verschieden durch Hydroxy substituiert sind,
oder
- R³ und R⁴: gemeinsam mit dem Stickstoffatom einen Piperidinyl-, Morpholinyhing oder einen Rest der Formel 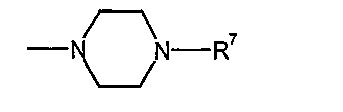 worin
R⁷ Wasserstoff, Methyl oder Ethyl bedeutet, das gegebenenfalls ein- bis zweifach, gleich oder verschieden durch Hydroxy, Methoxy oder Ethoxy substituiert ist, oder Cyclopentyl oder Cyclohexyl bedeutet,
und die unter R³ und R⁴ aufgeführten, gemeinsam mit dem Stickstoffatom gebildeten Heterocyclen, sind gegebenenfalls ein- bis zweifach, gleich oder verschieden, gegebenenfalls auch geminal, durch Hydroxy, Methyl oder Ethyl substituiert,
R⁵ und R⁶ gleich oder verschieden voneinander Wasserstoff, geradkettiges oder verzweigtes Alkyl mit bis zu 6 Kohlenstoffatomen, Hydroxy oder für geradkettiges oder verzweigtes Alkoxy mit bis zu 6 Kohlenstoffatomen.

Gemäß einer besonders bevorzugten Ausführungsform der vorliegenden Erfindung bedeuten bei den Reaktanden und dem Endprodukt des erfindungsgemäßen Verfahrens
- R¹: Methyl oder Ethyl,
- R²: n-Propyl,
- R³ und R⁴: gleich oder verschieden voneinander Methyl oder Ethyl, die gegebenenfalls bis zu zweifach gleich oder verschieden durch Hydroxy substituiert sind,
oder
- R³ und R⁴: gemeinsam mit dem Stickstoffatom einen Piperidinyl-, Morpholinylring oder einen Rest der Formel 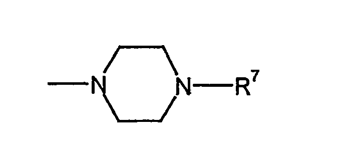 worin
R⁷ Wasserstoff, Methyl oder Ethyl bedeutet, das gegebenenfalls ein- bis zweifach, gleich oder verschieden durch Hydroxy, Methoxy oder Ethoxy substituiert ist, oder Cyclopentyl oder Cyclohexyl bedeutet,
und die unter R³ und R⁴ aufgeführten, gemeinsam mit dem Stickstoffatom gebildeten Heterocyclen, sind gegebenenfalls ein- bis zweifach, gleich oder verschieden, gegebenenfalls auch geminal, durch Hydroxy, Methyl oder Ethyl substituiert,
R⁵ Wasserstoff,
R⁶ Ethoxy.

Im Rahmen der vorliegenden Erfindung haben die Substituenten soweit nicht anders angegeben im allgemeinen die folgende Bedeutung:

Alkyl steht im allgemeinen für einen geradkettigen oder verzweigten Kohlenwasserstoffrest mit 1 bis 6 Kohlenstoffatomen. Beispielsweise seien Methyl, Ethyl, Propyl, Isopropyl, Butyl, Isobutyl, Pentyl, Isopentyl, Hexyl, Isohexyl genannt.

Alkoxy steht im allgemeinen für einen über einen Sauerstoffatom gebundenen geradkettigen oder verzweigten Kohlenwasserstoffrest mit 1 bis 6 Kohlenstoffatomen. Beispielsweise seien Methoxy, Ethoxy, Propoxy, Isopropoxy, Butoxy, Isobutoxy, Pentoxy Isopentoxy, Hexoxy, Isohexoxy genannt. Die Begriffe "Alkoxy" und "Alkyloxy" werden synonym verwendet.

Acyl steht im allgemeinen für geradkettiges oder verzweigtes Niedrigalkyl mit 1 bis 6 Kohlenstoffatomen, das über eine Carbonylgruppe gebunden ist. Beispielsweise seien genannt: Acetyl, Ethylcarbonyl, Propylcarbonyl, Isopropylcarbonyl, Butylcarbonyl und Isobutylcarbonyl.

Alkoxycarbonyl kann beispielsweise durch die Formel 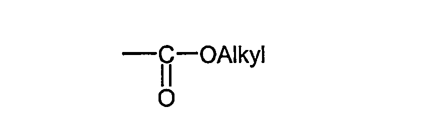 dargestellt werden.

Alkyl steht hierbei im allgemeinen für einen geradkettigen oder verzweigten Kohlenwasserstoffrest mit 1 bis 6 Kohlenstoffatomen. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Cycloalkyl steht im allgemeinen für einen cyclischen Kohlenwasserstoffrest mit 3 bis 8 Kohlenstoffatomen. Bevorzugt sind Cyclopropyl, Cyclopentyl und Cyclohexyl. Beispielsweise seien Cyclopentyl, Cyclohexyl, Cycloheptyl und Cyclooctyl genannt.

Halogen steht im Rahmen der Erfindung für Fluor, Chlor, Brom und Iod.

Als Lösungsmittel für die einzelnen Schritte des erfindungsgemäßen Verfahrens eignen sich die üblichen organischen Lösungsmittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösungsmittel zu verwenden.

Besonders bevorzugt wird die Umsetzung der Verbindungen der Formel (II) mit Schwefelsäure ohne Lösungsmittel und die anschließende Eintopfreaktion bei der Umsetzung mit Thionylchlorid vorzugsweise ohne Lösungsmittel und bei der anschließenden Reaktion mit dem Amin der Formel (IV) in Xylol durchgeführt.

Die Reaktionstemperatur kann bei den erfindungsgemäßen Verfahrensschritten im allgemeinen in einem größeren Bereich variiert werden. Im allgemeinen arbeitet man in einem Bereich von -20°C bis 200°C, bevorzugt von 0°C bis 70°C.

Die erfindungsgemäßen Verfahrensschritte werden im allgemeinen bei Normaldruck durchgeführt. Es ist aber auch möglich, sie bei Überdruck oder bei Unterdruck durchzuführen (z.B. in einem Bereich von 0,5 bis 5 bar).

Im ersten Schritt des erfindungsgemäßen Verfahrens wird die Schwefelsäure im Überschuss, beispielsweise im 2-20-fachen Überschuss, vorzugsweise im 2-10-fachen Überschuss jeweils bezogen auf 1 mol der Verbindung der Formel (II) eingesetzt.

Im zweiten Schritt des erfindungsgemäßen Verfahrens wird das Thionylchlorid im Überschuss, beispielsweise im 2-20-fachen Überschuss, vorzugsweise im 5-15-fachen Überschuss jeweils bezogen auf 1 mol der Verbindung der Formel (III) eingesetzt. Das

Amin (IV) wird äquimolar oder im Überschuss, beispielsweise im 2-10-fachen Überschuss, vorzugsweise im 2-5-fachen Überschuss jeweils bezogen auf 1 mol der Verbindung der Formel (III) eingesetzt.

Die Umsetzung der Verbindung der Formel (III) mit Thionylchlorid erfolgt vorzugsweise in Gegenwart katalytischer Mengen einer Base, wobei unter katalytischen Mengen ein deutlicher (beispielsweise mindestens zehnfacher) Unterschuss der Base im Vergleich zu den Reaktanden zu verstehen ist. Als Basen eignen sich im allgemeinen cyclische Amine, wie beispielsweise Piperidin, Pyridin, 4-N,N-Dimethylaminopyridin, oder C₁-C₄-Alkylamine, wie beispielsweise Triethylamin, oder bevorzugt Amide wie beispielsweise N,N-Dimethylformamid oder N,N-Dibutylformamid. Besonders bevorzugt ist N,N-Dimethylformamid.

Die Verbindungen der Formel (II) können wie in der WO 99/24433 beschrieben hergestellt werden aus Verbindungen der Formel (V) 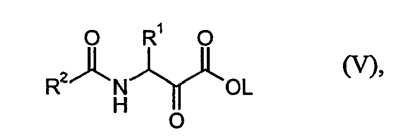 in welcher
R¹ und R² die oben angegebene Bedeutung haben
und
- L: für geradkettiges oder verzweigtes Alkyl mit bis zu 4 Kohlenstoffatomen steht,
mit Verbindungen der allgemeinen Formel (VI) 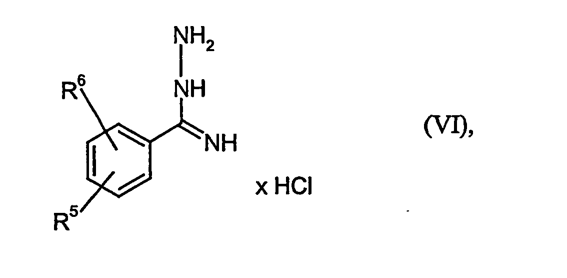 in welcher

R⁵ und R⁶ die oben angegebene Bedeutung haben.

Diese Reaktion kann entweder wie in der WO 99/24433 beschrieben in einer Zweistufenreaktion im System Ethanol und Phosphoroxytrichlorid/Dichlorethan oder vorzugsweise gemäß der vorliegenden Erfindung in einer Zweistufenreaktion als Eintopfverfahren in den Systemen Methanol und Phosphoroxytrichlorid/Essigsäure oder besonders bevorzugt Methanol und Acetylchlorid/Essigsäure durchgeführt werden.

Als Lösungsmittel für diese Reaktion eignen sich die üblichen organischen Lösungsmittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethan, Trichlorethylen oder Chlorbenzol, oder Alkohole wie Methanol, Ethanol, n-Propanol, Isopropanol oder n-Butanol, oder Essigester, Acetonitril, Aceton, Dimethoxyethan oder Pyridin oder Säuren wie Essigsäure. Ebenso ist es möglich, Gemische der genannten Lösungsmittel zu verwenden. Bevorzugt sind gemäß der vorliegenden Erfindung für den ersten Schritt Alkohole, besonders bevorzugt Methanol, und für den zweiten Schritt entweder Dichlorethan (wie in der WO 99/24433 beschrieben) oder besonders bevorzugt Essigsäure.

Die Reaktionstemperatur kann bei dieser Reaktion im allgemeinen in einem größeren Bereich variiert werden. Im allgemeinen arbeitet man in einem Bereich von -20°C bis 200°C, bevorzugt von 0°C bis 70°C.

Diese Reaktion wird im allgemeinen bei Normaldruck durchgeführt. Es ist aber auch möglich, die Reaktion bei Überdruck oder bei Unterdruck durchzuführen (z.B. in einem Bereich von 0,5 bis 5 bar).

Die Reaktanden werden bei dieser Reaktion als Rohprodukte eingesetzt. Je nach Beschaffenheit der Reaktanden können diese in äquimolaren Mengen oder einer der beiden Reaktanden im Überschuss eingesetzt werden.

Die Verbindungen der Formel (V) sind teilweise bekannt oder können gemäß der WO 99/24433 hergestellt werden, indem man Verbindungen der allgemeinen Formel (VII)

R²-CO-T (VII)

in welcher
- R²: die oben angegebene Bedeutung hat
und
- T: für Halogen, vorzugsweise für Chlor steht,
zunächst durch Umsetzung mit Verbindungen der allgemeinen Formel (VIII) 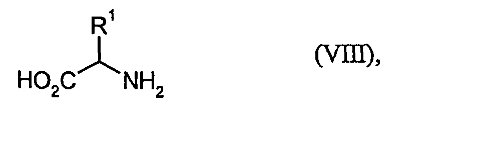 in welcher
- R¹: die oben angegebene Bedeutung hat

gegebenenfalls in inerten Lösungsmitteln, gegebenenfalls in Anwesenheit einer Base und/oder Trimethylsilylchlorid in die Verbindungen der allgemeinen Formel (IX) 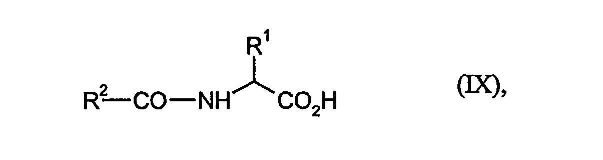 in welcher
R¹ und R²die oben angegebene Bedeutung haben,
überführt und abschließend mit der Verbindung der Formel (X) 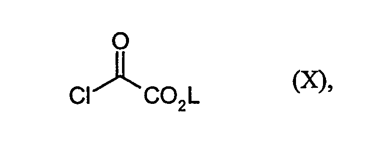 worin
L die oben angegebene Bedeutung hat,
in inerten Lösungsmitteln, gegebenenfalls in Anwesenheit einer Base umsetzt.

Als Lösungsmittel für die einzelnen Schritte des Verfahrens eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Dimethylformamid, Hexamethylphosphorsäuretriamid, Acetonitril, Aceton, Dimethoxyethan oder Pyridin. Ebenso ist es möglich, Gemische der genannten Lösungsmittel zu verwenden.

Besonders bevorzugt ist für den ersten Schritt, der Umsetzung der Verbindung der Formel (VII) mit der Verbindung der Formel (VIII), die Durchführung der Reaktion in Dichlormethan oder die Durchführung der Umsetzung ohne inertes Lösungsmittel, wobei man in Gegenwart einer Base, vorzugsweise einem Alkali- oder Erdalkalimetallhydroxid, besonders bevorzugt Natronlauge, als Lösungsmittel arbeitet.

Besonders bevorzugt ist für den zweiten Schritt, der Umsetzung der Verbindung der Formel (IX) mit der Verbindung der Formel (X), die Durchführung der Reaktion in einem Gemisch aus Tetrahydrofuran und Pyridin.

Als Basen eignen sich im allgemeinen cyclische Amine, wie beispielsweise Piperidin, Pyridin, Dimethylaminopyridin oder C₁-C₄-Alkylamine, wie beispielsweise Triethylamin. Bevorzugt sind Triethylamin, Pyridin und/oder Dimethylaminopyridin.

Die Base wird im allgemeinen in einer Menge von 1 bis 5 Mol-Äquivalenten, bevorzugt 1 bis 3 Mol-Äquivalenten im Vergleich zu den Reaktanden eingesetzt.

Die Reaktionstemperatur kann im allgemeinen in einem größeren Bereich variiert werden. Im allgemeinen arbeitet man in einem Bereich von -20°C bis 200°C, bevorzugt von 0°C bis 100°C.

Die Verbindungen der Formel (VII), (VIII) und (X) sind an sich bekannt oder nach dem Fachmann bekannten Methoden herstellbar, beispielsweise gemäß den in der WO 99/24433 beschriebenen Verfahren.

Die Verbindungen der Formel (VII) und (VIII) werden je nach Beschaffenheit der Reaktanden in äquimolaren Mengen oder einer der beiden Reaktanden im Überschuss eingesetzt.

Die Verbindungen der Formel (IX) und (X) werden je nach Beschaffenheit der Reaktanden in äquimolaren Mengen oder einer der beiden Reaktanden im Überschuss eingesetzt. Vorzugsweise wird die Verbindung der Formel (X) im 1,5- bis 5-fachen Überschuss eingesetzt.

Die Verbindungen der Formel (VI) können auf verschiedenen Wegen hergestellt werden. Beispielsweise können sie gemäß der WO 99/24433 hergestellt werden, indem man Verbindungen der allgemeinen Formel (XI) 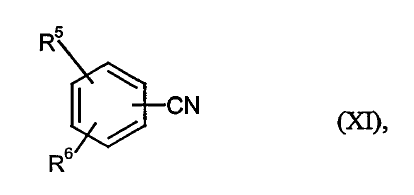 in welcher
R⁵ und R⁶ die oben angegebene Bedeutung haben,
mit Ammoniumchlorid in Toluol und in Anwesenheit von Trimethylaluminium in Hexan in einem Temperaturbereich von -20°C bis Raumtemperatur, vorzugsweise bei 0°C und Normaldruck umsetzt und das entstehende Amidin, gegebenenfalls in situ, mit Hydrazinhydrat umsetzt.

Die Verbindungen der allgemeinen Formel (XI) sind an sich bekannt oder nach üblichen Methoden herstellbar. Beispielsweise sind diese gemäß der WO 99/24433 aus den entsprechenden Phenolderivaten durch Veretherung zugänglich. Möglich ist aber auch die Umsetzung der entsprechenden Benzamide in einem inerten organischen Lösungsmittel wie Toluol mit Thionylchlorid unter Erhitzen auf beispielsweise 50-100°C, vorzugsweise 70-100°C zu den Verbindungen der Formel (XI).

Alternativ können die Verbindungen der Formel (VI) aber auch hergestellt werden, indem man die Verbindungen der Formel (XI) in Gegenwart einer Base in einem inerten organischen Lösungsmittel mit Hydroxylamin-Hydrochlorid zu den Verbindungen der Formel (XII) 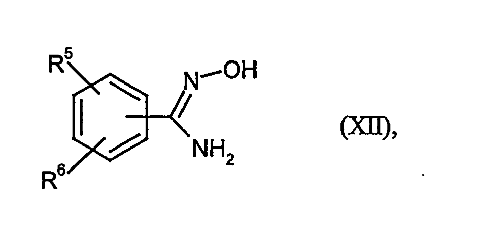 worin
R⁵ und R⁶ die oben angegebene Bedeutung haben,
und anschließend mit einem Reduktionsmittel in einem organischen Lösungsmittel zu den Verbindungen der Formel (XIII) 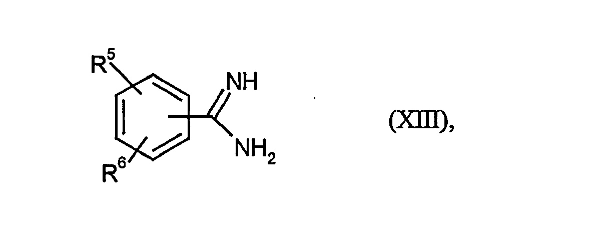 worin
R⁵ und R⁶ die oben angegebene Bedeutung haben,
umsetzt, die dann durch Reaktion mit Hydrazinhydrat gegebenenfalls in situ zu den Verbindungen der Formel (VI) umgesetzt werden können.

Als Lösungsmittel für diese Reaktionen eignen sich die üblichen organischen Lösungsmittel, die sich unter den Reaktionsbedingungen nicht verändern. Hierzu gehören bevorzugt Alkohole wie Methanol, Ethanol oder iso-Propanol, Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethan, Trichlorethylen oder Chlorbenzol, oder Essigester, Dimethylformamid, Hexamethylphosphorsäuretriamid, Acetonitril, Aceton, Dimethoxyethan oder Pyridin oder Essigsäure. Ebenso ist es möglich, Gemische der genannten Lösungsmittel zu verwenden.

Besonders bevorzugt wird die Umsetzung der Verbindungen der Formel (XI) zu den Verbindungen der Formel (XII) in Isopropanol durchgeführt. Besonders bevorzugt wird die Umsetzung der Verbindungen der Formel (XII) zu den Verbindungen der Formel (XIII) in Essigsäure durchgeführt. Die Umsetzung der Verbindung der Formel (XIII) mit Hydrazinhydrat erfolgt besonders bevorzugt in Methanol.

Diese Reaktionen werden im allgemeinen bei Normaldruck durchgeführt. Es ist aber auch möglich, bei Überdruck oder bei Unterdruck zu arbeiten (z.B. in einem Bereich von 0,5 bis 5 bar).

Die Reaktionstemperatur kann im allgemeinen in einem größeren Bereich variiert werden. Im allgemeinen arbeitet man in einem Bereich von -20°C bis 200°C, bevorzugt von 0°C bis 100°C.

Die Umsetzung der Verbindungen der Formel (XI) zu den Verbindungen der Formel (XII) erfolgt in Gegenwart einer Base. Als Basen eignen sich insbesondere cyclische Amine, wie beispielsweise Piperidin, Pyridin, Dimethylaminopyridin oder C₁-C₄₋Alkylamine, wie beispielsweise Triethylamin. Bevorzugt ist Triethylamin. Die Base wird im allgemeinen in einer Menge von 1 mol bis 4 mol jeweils bezogen auf 1 mol der Verbindung der Formel (XI) eingesetzt.

Die Reduktion der Verbindungen der Formel (XII) zu den Verbindungen der Formel (XIII) kann mit den für derartige Reaktionen üblichen Reduktionsmitteln unter den dem Fachmann bekannten Bedingungen durchgeführt werden. Erfindungsgemäß bevorzugt ist die Hydrierung in Gegenwart eines Katalysators wie Palladium auf Kohle in Essigsäure.

Je nach Beschaffenheit der Reaktanden werden die Verbindungen der Formel (XI) und Hydroxylamin-Hydrochlorid beziehungsweise die Verbindungen der Formel (XIII) und Hydrazinhydrat und in äquimolaren Mengen oder das Hydroxylamin-Hydrochlorid beziehungsweise das Hydrazinhydrat im Überschuss eingesetzt.

Die erfindungsgemäßen Verbindungen sind Inhibitoren cGMP metabolisierender PDE's und bereits in der WO 99/24433 beschrieben.

Die vorliegende Erfindung wird nachstehend anhand von nicht einschränkenden bevorzugten Beispielen und Vergleichsbeispielen näher dargestellt. Soweit nicht anderweitig angegeben, beziehen sich alle Mengenangaben auf Gewichtsprozente.

### Beispiele

¹H-NMR-Spektren wurden mit dem Spektrometer WP-200 SY der Firma Bruker bei Raumtemperatur gemessen. Als Lösungsmittel dienten deuteriertes Dimethylsulfoxid oder Deuterochloroform mit Tetramethylsilan als internem Standard (falls nicht anders vermerkt).

MS-Spektren wurden mit den Spektrometern M 40 der Firma AMD und API 150 der Firma PE/SCIEX gemessen. Angegeben wird die relative Signalintensität (in Prozent bezogen auf den Basispeak).

HPLC-Analytik wurde mit dem Gerät HP 1090 der Firma Hewlett Packard aufgenommen. Die genauen Bedingungen sind bei den jeweiligen Ausführungsbeispielen angegeben.

### Ausgangsverbindungen

### Beispiel I: Herstellung von 2-(2-Ethoxy-phenyl)-5-methyl-7-propyl-3H-imidazo-[5,1-f][1,2,4]triazin-4-on

### Ia) Herstellung von 2-Butyrylaminopropionsäure

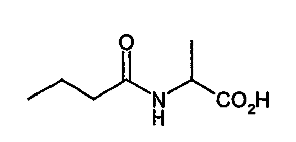

Eine Lösung von 100 kg D,L-Alanin in wässeriger Natronlauge wird in der Kälte mit 119 kg Buttersäurechlorid umgesetzt. Nach Zugabe von Butylacetat wird mit Salzsäure angesäuert, die organische Phase abgetrennt und die wässerige Phase nachextrahiert. Die organische Phase wird durch azeotrope Destillation getrocknet. Das Kristallisat wird isoliert, mit Butylacetat gewaschen und getrocknet.
- Ausbeute:: 132.6 kg (68%)
- ¹H-NMR:: δ = 0.8 (t, 3 H), 1.25 (d, 3 H), 1.5 (m, 2 H), 2.1 (t, 2 H), 4.2 (q, 1 H), 8.1 (d, NH), 12.0-12.7 (s, COOH)
- MS:: 336 (2M+NH₄, 40), 319 (2M+H, 15), 177 (M+NH₄, 100), 160 (M+H, 20)

### Ib) Herstellung von 2-Ethoxybenzonitril

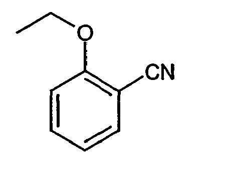

Zu einer Suspension von 250 kg 2-Ethoxybenzamid in Toluol wird bei 85-95°C 260 kg Thionylchlorid dosierkontrolliert zugegeben. Die Reaktionsmischung wird in der Wärme nachgerührt. Thionylchlorid und Toluol werden anschließend im Vakuum abdestilliert. Das Produkt wird als Rohprodukt in die Folgestufe eingesetzt.
- Ausbeute:: 228.5 kg (Rohprodukt)
- ¹H-NMR:: δ = 1.45 (t, 3 H), 4.15 (q, 2 H), 7.0 (m, 2 H, Phenyl), 7.5 (m, 2 H, Phenyl)
- MS:: 312 (2M+NH₄, 35), 165 (M+NH₄, 100), 147 (5)

### Ic) Herstellung von 2-Ethoxy-N-hydroxybenzamidin

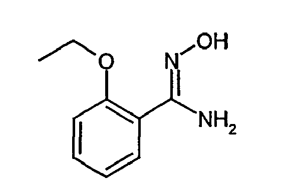

111 kg 2-Ethoxybenzonitril (Rohprodukt) aus Beispiel Ib werden mit 1641 Triethylamin und 73 kg Hydroxylaminhydrochlorid in Isopropanol unter Rückfluss erhitzt. Die Reaktionsmischung wird mit Wasser versetzt und abgekühlt. Das Kristallisat wird isoliert, gewaschen und als Feuchtprodukt in die Folgestufe eingesetzt.
- Ausbeute:: 92.6 kg (Feuchtprodukt)
- ¹H-NMR:: δ = 1.35 (t, 3 H), 4.1 (q, 2 H), 5.6 (s, 2 H), 6.9-7.4 (4 H, Phenyl), 9.4 (s, 1 H, OH)
- MS:: 361 (2M+H, 30), 198 (M+NH₄, 30), 181 (M+H, 100)

### Id) Herstellung von 2-Ethoxybenzamidin-Hydrochlorid

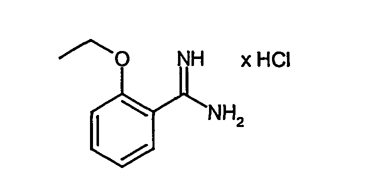

135 kg 2-Ethoxy-N-hydroxybenzamidin (Feuchtprodukt) aus Beispiel Ic werden in Essigsäure mit Palladium auf Kohle als Katalysator bei 50-60°C hydriert. Zur Aufarbeitung wird die Hydrierreaktion vom Katalysator befreit, mit Salzsäure versetzt und eingeengt. Restliche Essigsäure und Wasser werden durch azeotrope Destillation mit Toluol entfernt. Das Kristallisat wird isoliert und im Vakuum getrocknet.
- Ausbeute:: 136.4 kg
- H-NMR:: 1.35 (t, 3 H), 4.15 (q, 2 H), 7.1-7.7 (m, 4 H, Phenyl), 9.1-9.4 (2 x s, 3 H), 10.5-10.7 (s, 1 H)
- MS:: 329 (2M+H, 10), 165 (M+H, 100)

### Ie) Herstellung von 2-(2-Ethoxy-phenyl)-5-methyl-7-propyl-3H-imidazo[5,1-ƒ][1,2,4]triazin-4-on

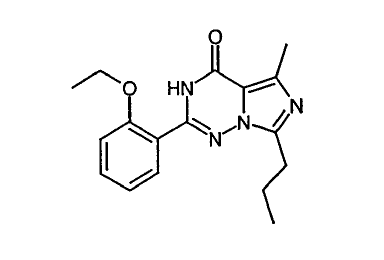

231 kg 2-Butyrylaminopropionsäure aus Beispiel Ia werden in Tetrahydrofuran mit 341 kg Pyridin, katalytischen Mengen 4-N,N-Dimethylaminopyridin und 392 kg Ethylchloroxalat versetzt und unter Erhitzen unter Rückfluss nachgerührt. Die Reaktionsmischung wird in Ethylacetat aufgenommen, mit Wasser gewaschen und die Ethylacetat-Phase eingeengt. Der Destillationsrückstand wird in Methanol aufgenommen und mit der folgenden Lösung umgesetzt.

192 kg 2-Ethoxybenzamidin-Hydrochlorid aus Beispiel Id wird in Methanol mit 47.5 kg Hydrazinhydrat versetzt und bei Raumtemperatur nachgerührt. Die Lösung wird mit der vorstehend hergestellten Lösung des Oxalsäure-2-butyrylamino-1-ethoxy-carbonyl-propenylesterethylesters vereinigt. Die so erhaltene Reaktionsmischung wird unter Erhitzen unter Rückfluss nachgerührt. Methanol wird destillativ entfernt und durch Essigsäure ersetzt.

### Option A:

Es wird 138.6 kg Phosphoroxychlorid zugesetzt und in der Wärme nachgerührt. Essigsäure wird im Vakuum abdestilliert. Der Rückstand wird mit Wasser und Dichlormethan oder optional Methylisobutylketon versetzt und mit Natronlauge neutral gestellt. Die organische Phase wird eingeengt, und der Rückstand wird in Aceton gelöst und unter Abkühlen kristallisiert. Das Kristallisat wird isoliert, gewaschen und getrocknet.

### Option B:

Es wird mindestens 190 kg Acetylchlorid zugesetzt und in der Wärme nachgerührt. Essigsäure wird im Vakuum abdestilliert. Der Destillationsrückstand wird mit Aceton und Wasser versetzt, und das Produkt wird durch Neutralstellen mit Natronlauge kristallisiert. Das Produkt wird isoliert, gewaschen und getrocknet.
- Ausbeute:: 90-160 kg
- ¹H-NMR:: δ = 1.0 (t, 3 H), 1.6 (t, 3 H), 1.9 (m, 2 H), 2.8 (s, 3 H), 3.3 (t, 2 H), 4.3 (q, 2 H), 7.0-8.2 (Ar, 4 H), 10.3 (CONH, 1 H)
- MS:: 313 (M+H, 100), 149 (25), 151 (40), 121 (15)
- HPLC:: Kromasil C-18-Phase, neutraler Phosphatpuffer, Acetonitril, 233 nm, linearer Gradient 30% Acetonitril -> 80% Acetonitril (30 min.): 99 F1.%(Rₜ 19.1)

### Herstellungsbeispiele

### Beispiel 1a: 4-Ethoxy-3-(5-methyl-4-oxo-7-propyl-3,4-dihydro-imidazo[5,1-f][1,2,4]triazin-2-yl)-benzolsulfonsäure

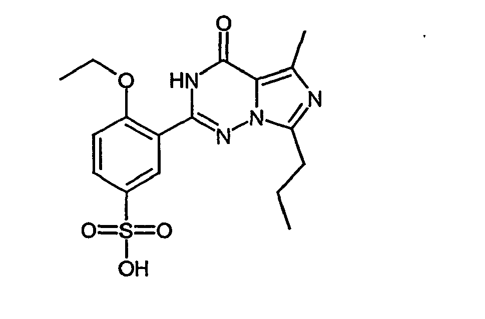

194 kg 2-(2-Ethoxy-phenyl)-5-methyl-7-propyl-3H-imidazo[5,1-f][1,2,4]triazin-4-on aus Beispiel Ie wird mit 504 kg konzentrierter Schwefelsäure umgesetzt. Die Reaktionsmischung wird auf Wasser gegeben, abgekühlt, und das Kristallisat wird isoliert und im Vakuum getrocknet.
- Ausbeute:: 195.2 kg
- ¹H-NMR:: δ = 0.95 (t, 3 H), 1.3 (t, 3 H), 1.8 (m, 2 H), 2.6 (s, 3 H), 3.05 (t, 2 H), 4.1 (q, 2 H), 7.15 (Ar, 1 H), 7.75 (m, 2 H), 12.3 (SO₂OH)
- MS:: 393 (M+H, 100), 365 (25), 151 (40)
- HPLC:: X-Terra C-18-Phase, wässrige Phosphorsäure, Acetonitril, 242 nm, linearer Gradient 10% Acetonitril -> 90% Acetonitril (20 min.): 98 F1.% (Rₜ 9.2)

### Beispiel 1b): 2-[2-Ethoxy-5-(4-ethyl-piperazin-1-sulfonyl)-phenyl]-5-methyl-7-propyl-3H-imidazo[5,1-f][1,2,4]triazin-4-on

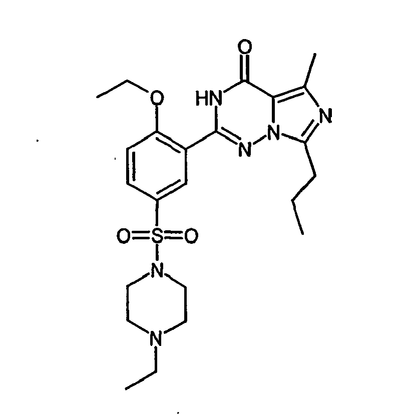

22.5 kg 4-Ethoxy-3-(5-methyl-4-oxo-7-propyl-3,4-dihydro-imidazo[5,1-f][1,2,4]triazin-2-yl)-benzolsulfonsäure aus Beispiel 1a wird mit 74 kg Thionylchlorid und katalytischen Mengen Dimethylformamid bis zum Ende der Gasentwicklung umgesetzt. Zu der Reaktionsmischung wird wiederholt Xylol gegeben und Thionylchlorid abdestilliert. Zu der Suspension wird 15.1 kg N-Ethylpiperazin gegeben und nachgerührt. Nach der Zugabe von Wasser wird mit Salzsäure auf pH 1 eingestellt, und die Phasen werden getrennt. Die wässerige Phase wird mit Aceton versetzt und durch Zugabe von Natronlauge neutral gestellt. Die Mischung wird abgekühlt, und das Kristallisat wird isoliert, gewaschen und im Vakuum getrocknet.
- Ausbeute:: 26.1 kg
- ¹H-NMR:: δ = 1.0 (2 x t, 6 H), 1.6 (t, 3 H), 1.9 (m, 2 H), 2.45 (q, 2 H), 2.55 (m, 4 H), 2.65 (s, 3 H), 3.0 (t, 2 H), 3.1 (m, 4 H), 4.35 (q, 2 H), 7.15 (Ar, 1 H), 7.9 (Ar, 1 H), 8.4 (Ar, 1 H), 9.8 (CONH)
- MS:: 489 (M+H, 100), 345 (10), 313, (10), 285 (10), 113 (20)
- HPLC:: X-Terra C-18-Phase, neutraler Phosphatpuffer, Acetonitril, 242 nm, linearer Gradient 20% Acetonitril -> 75% Acetonitril (20 min.): 98 Fl.% (Rₜ 16.3)

### 1c) 2-[2-Ethoxy-5-(4-ethyl-piperazin-1-sulfonyl)-phenyl]-5-methyl-7-propyl-3H imidazo[5,1-f][1,2,4]triazin-4-on-Hydrochlorid-Trihydrat

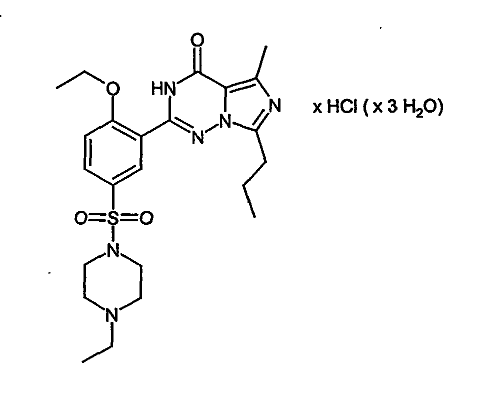

22.5 kg 2-[2-Ethoxy-5-(4-ethyl-piperazin-1-sulfonyl)-phenyl]-5-methyl-7-propyl-3H-imidazo[5,1-f][1,2,4]triazin-4-on aus Beispiel 1b wird in 5.1 kg konzentrierter Salzsäure und Aceton/Wasser (12:1 v/v) in der Wärme aufgelöst. Die klare Lösung wird heiß filtriert und durch Abkühlen und Animpfen kristallisiert. Das Kristallisat wird isoliert, gewaschen und im Vakuum bei ca. 30°C und ca. 300 mbar getrocknet.
- Ausbeute:: 25.4 kg
- Smp. (DSC):: 192°C
- HPLC:: X-Terra C-18-Phase, neutraler Phosphatpuffer, Acetonitril, 242 nm, linearer Gradient 20% Acetonitril -> 75% Acetonitril (20 min.): 99 F1.% (Rₜ 16.3)

## Patentansprüche

1. Verfahren zur Herstellung von Verbindungen der Formel (I) 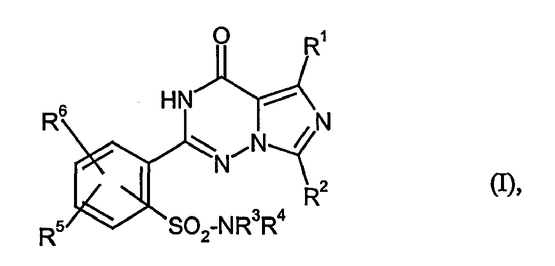 in welcher
R¹ für Wasserstoff oder geradkettiges oder verzweigtes Alkyl mit bis zu 4 Kohlenstoffatomen steht,
R² für geradkettiges Alkyl mit bis zu 4 Kohlenstoffatomen steht,
R³ und R⁴ gleich oder verschieden sind und für eine geradkettige oder verzweigte Alkylkette mit bis zu 5 Kohlenstoffatomen stehen, die gegebenenfalls bis zu zweifach gleich oder verschieden durch Hydroxy oder Methoxy substituiert ist,
oder
R³ und R⁴ gemeinsam mit dem Stickstoffatom einen Piperidinyl-, Morpholinyl-, Thiomorpholinylring oder einen Rest der Formel 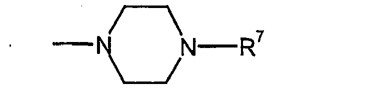 bilden,
worin
R⁷ Wasserstoff, Formyl, geradkettiges oder verzweigtes Acyl oder Alkoxycarbonyl mit jeweils bis zu 6 Kohlenstoffatomen bedeutet, oder geradkettiges oder verzweigtes Alkyl mit bis zu 6 Kohlenstoffatomen bedeutet, das gegebenenfalls ein- bis zweifach, gleich oder verschieden durch Hydroxy, Carboxyl, geradkettiges oder verzweigtes Alkoxy oder Alkoxycarbonyl mit jeweils bis zu 6 Kohlenstoffatomen substituiert ist, oder C₃₋8-Cycloalkyl bedeutet,
und die unter R³ und R⁴ aufgeführten, gemeinsam mit dem Stickstoffatom gebildeten Heterocyclen, gegebenenfalls ein- bis zweifach, gleich oder verschieden, gegebenenfalls auch geminal, durch Hydroxy, Formyl, Carboxyl, geradkettiges oder verzweigtes Acyl oder Alkoxycarbonyl mit bis jeweils zu 6 Kohlenstoffatomen substituiert sind,
und/oder die unter R³ und R⁴ aufgeführten, gemeinsam mit dem Stickstoffatom gebildeten Heterocyclen, gegebenenfalls durch geradkettiges oder verzweigtes Alkyl mit bis zu 6 Kohlenstoffatomen substituiert sind, das gegebenenfalls ein- bis zweifach, gleich oder verschieden durch Hydroxy, Carboxyl substituiert ist,
und/oder die unter R³ und R⁴ aufgeführten, gemeinsam mit dem Stickstoffatom gebildeten Heterocyclen, gegebenenfalls durch über N-verknüpftes Piperidinyl oder Pyrrolidinyl substituiert sind,
R⁵ und R⁶ gleich oder verschieden sind und für Wasserstoff, geradkettiges oder verzweigtes Alkyl mit bis zu 6 Kohlenstoffatomen, Hydroxy oder für geradkettiges oder verzweigtes Alkoxy mit bis zu 6 Kohlenstoffatomen stehen,
**dadurch gekennzeichnet, dass** Verbindungen der Formel (II) 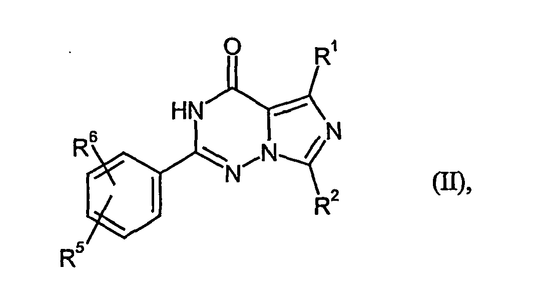 worin
R¹, R², R⁵ und R⁶ die vorstehend angegebenen Bedeutungen haben,
mit Schwefelsäure zu Verbindungen der Formel (III) 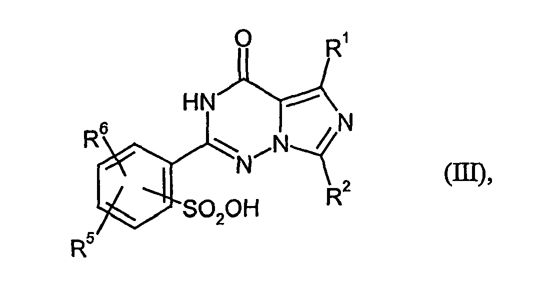 worin
R¹, R², R⁵ und R⁶ die vorstehend angegebenen Bedeutungen haben,
und anschließend mit Thionylchlorid und das so erhaltene Produkt in situ in einem inerten Lösungsmittel mit einem Amin der Formel (TV) 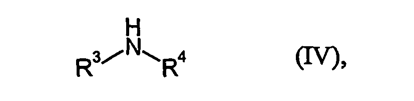 worin
R³ und R⁴ die vorstehend angegebene Bedeutung haben,
sowie gegebenenfalls zu den entsprechenden Salzen, Hydraten oder N-Oxiden
umgesetzt werden.

2. Verfahren nach Anspruch 1, **dadurch gekennzeichnet, dass**
R¹ für Wasserstoff oder geradkettiges oder verzweigtes Alkyl mit bis zu 4 Kohlenstoffatomen steht,
R² für geradkettiges Alkyl mit bis zu 4 Kohlenstoffatomen steht,
R³ und R⁴ gleich oder verschieden sind und für eine geradkettige oder verzweigte Alkylkette mit bis zu 5 Kohlenstoffatomen stehen, die gegebenenfalls bis zu zweifach gleich oder verschieden durch Hydroxy oder Methoxy substituiert ist,
oder
R³ und R⁴ gemeinsam mit dem Stickstoffatom einen Piperidinyl-, Morpholinylring oder einen Rest der Formel 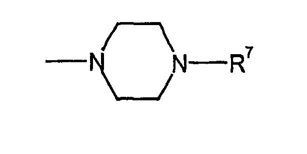 bilden,
worin
R⁷ Wasserstoff, geradkettiges oder verzweigtes Alkyl mit bis zu 4 Kohlenstoffatomen bedeutet, das gegebenenfalls ein- bis zweifach, gleich oder verschieden durch Hydroxy, geradkettiges oder verzweigtes Alkoxy mit jeweils bis zu 4 Kohlenstoffatomen substituiert ist, oder C₃₋₆-Cycloalkyl bedeutet,
und die unter R³ und R⁴ aufgeführten, gemeinsam mit dem Stickstoffatom gebildeten Heterocyclen, gegebenenfalls ein- bis zweifach, gleich oder verschieden, gegebenenfalls auch geminal, durch Hydroxy, geradkettiges oder verzweigtes Acyl oder Alkoxycarbonyl mit bis jeweils zu 4 Kohlenstoffatomen, gegebenenfalls durch geradkettiges oder verzweigtes Alkyl mit bis zu 4 Kohlenstoffatomen substituiert sind, das gegebenenfalls ein- bis zweifach, gleich oder verschieden durch Hydroxy substituiert ist,
R⁵ und R⁶ gleich oder verschieden sind und für Wasserstoff, geradkettiges oder verzweigtes Alkyl mit bis zu 6 Kohlenstoffatomen, Hydroxy oder für geradkettiges oder verzweigtes Alkoxy mit bis zu 6 Kohlenstoffatomen stehen.

3. Verfahren nach Anspruch 1, **dadurch gekennzeichnet, dass**
R¹ für Wasserstoff oder geradkettiges oder verzweigtes Alkyl mit bis zu 4 Kohlenstoffatomen steht,
R² für geradkettiges Alkyl mit bis zu 4 Kohlenstoffatomen steht,
R³ und R⁴ gleich oder verschieden sind und für Methyl oder Ethyl stehen, die gegebenenfalls bis zu zweifach gleich oder verschieden durch Hydroxy substituiert sind,
oder
R³ und R⁴ gemeinsam mit dem Stickstoffatom einen Piperidinyl-, Morpholinylring oder einen Rest der Formel 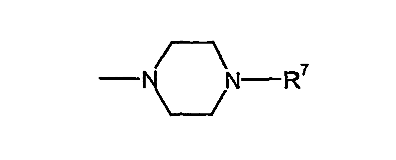 bilden,
worin
R⁷ Wasserstoff, Methyl oder Ethyl bedeutet, das gegebenenfalls ein- bis zweifach, gleich oder verschieden durch Hydroxy, Methoxy oder Ethoxy substituiert ist, oder Cyclopentyl oder Cyclohexyl bedeutet,
und die unter R³ und R⁴ aufgeführten, gemeinsam mit dem Stickstoffatom gebildeten Heterocyclen, gegebenenfalls ein- bis zweifach, gleich oder verschieden, gegebenenfalls auch geminal, durch Hydroxy, Methyl oder Ethyl substituiert sind,
R⁵ und R⁶ gleich oder verschieden sind und für Wasserstoff, geradkettiges oder verzweigtes Alkyl mit bis zu 6 Kohlenstoffatomen, Hydroxy oder für geradkettiges oder verzweigtes Alkoxy mit bis zu 6 Kohlenstoffatomen stehen.

4. Verfahren nach Anspruch 1, **dadurch gekennzeichnet, dass**
R¹ Methyl oder Ethyl bedeutet,
R² n-Propyl bedeutet,
R³ und R⁴ gleich oder verschieden sind und für Methyl oder Ethyl stehen, die gegebenenfalls bis zu zweifach gleich oder verschieden durch Hydroxy substituiert sind,
oder
R³ und R⁴ gemeinsam mit dem Stickstoffatom einen Piperidinyl-, Morpholinylring oder einen Rest der Formel 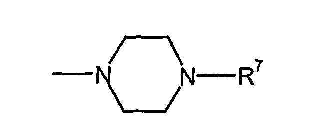 bilden,
worin
R⁷ Wasserstoff, Methyl oder Ethyl bedeutet, das gegebenenfalls ein- bis zweifach, gleich oder verschieden durch Hydroxy, Methoxy oder Ethoxy substituiert ist, oder Cyclopentyl oder Cyclohexyl bedeutet,
und die unter R³ und R⁴ aufgeftihrten, gemeinsam mit dem Stickstoffatom gebildeten Heterocyclen, gegebenenfalls ein- bis zweifach, gleich oder verschieden, gegebenenfalls auch geminal, durch Hydroxy, Methyl oder Ethyl substituiert sind,
R⁵ Wasserstoff bedeutet
R⁶ Ethoxy bedeutet.

5. Verfahren nach Anspruch 1, **dadurch gekennzeichnet, dass** die Verbindungen der Formel (II) durch Umsetzung der Verbindungen der Formel (V) 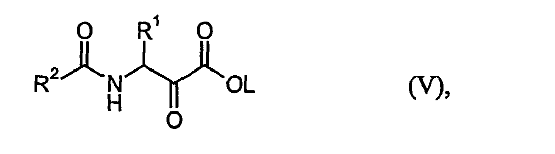 in welcher
R¹ und R² die in Anspruch 1 angegebene Bedeutung haben
L für geradkettiges oder verzweigtes Alkyl mit bis zu 4 Kohlenstoffatomen steht,
mit Verbindungen der allgemeinen Formel (VI) 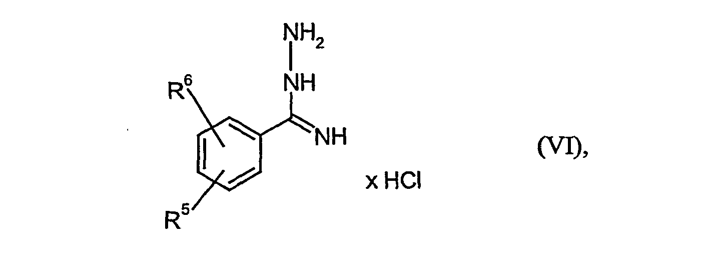 in welcher
R⁵ und R⁶ die in Anspruch 1 angegebene Bedeutung haben.
in einer Zweistufenreaktion in den Systemen Methanol und Phosphoroxytrichlorid/Essigsäure oder Methanol und Acetylchlorid/ Essigsäure durchgeführt werden.

## Claims

1. Process for the preparation of compounds of the formula I 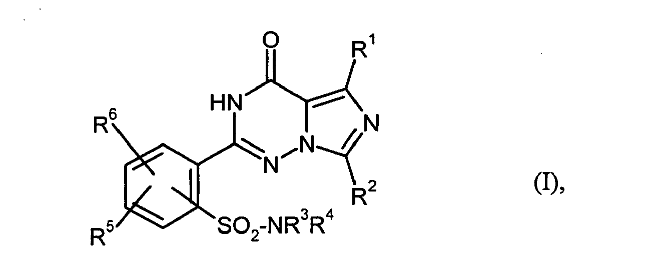 in which
R¹ represents hydrogen or straight-chain or brabched alkyl having up to 4 carbon atoms,
R² represents straight-chain alkyl having up to 4 carbon atoms,
R³ and R⁴ are identical or different and represent a straight-chain or branched alkyl chain having up to 5 carbon atoms, which is optionally substitued up to two times in an identical or different manner by hydroxyl or methyloxy,
or
R³ and R⁴, together with the nitrogen atom, from a piperidinyl, morpholinyl or thiomorpholinyl ring or a radical of the formula 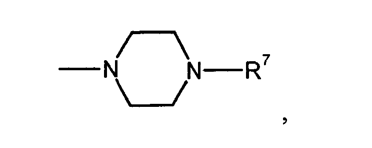 in which
R⁷ denotes hydrogen, formyl, straight-chain or branched acyl or alkoxycarbonyl each having up to 6 carbon atoms, or straight-chain or branched alkyl having up to 6 carbon atoms, which is optionally mono- to disubstituted, in an identical or different manner, by hydroxyl, carboxyl, straight-chain or branched alkoxy or alkoxycarbonyl each having up to 6 carbon atoms, or denotes C₃₋₈-cycloalkyl,
and the heterocycles mentioned under R³ and R⁴, formed together with the nitrogen atom, are optionally mono- to disubstituted, in an identical or different manner, if appropriate also geminally, by hydroxyl, formyl, carboxyl, straight-chain or branched acyl or alkoxycarbonyl each having up to 6 carbon atoms,
and/or the heterocycles mentioned under R³ and R⁴, formed together with the nitrogen atom, are optionally substituted by straight-chain or branched alkyl having up to 6 carbon atoms, which is optionally mono- to disubstituted, in an identical or different manner, by hydroxyl or carboxyl,
and/or the heterocycles mentioned under R³ and R⁴, formed together with the nitrogen atom, are optionally substituted by piperidinyl or pyrrolidinyl linked via N,
R⁵ and R⁶ are identical or different and represent hydrogen, straight-chain or branched alkyl having up to 6 carbon atoms, hydroxyl or straight-chain or branched alkoxy having up to 6 carbon atoms,
**characterized in that** compounds of the formula (II) 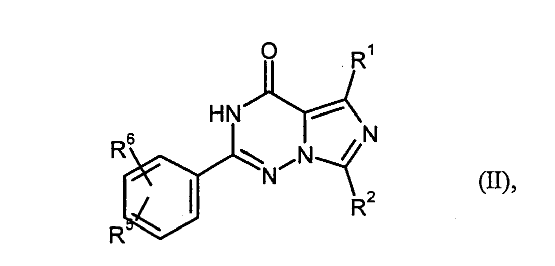 in which
R¹, R², R⁵ and R⁶ have the meanings indicated above,
are reacted with sulphuric acid to give compounds of the formula (III) 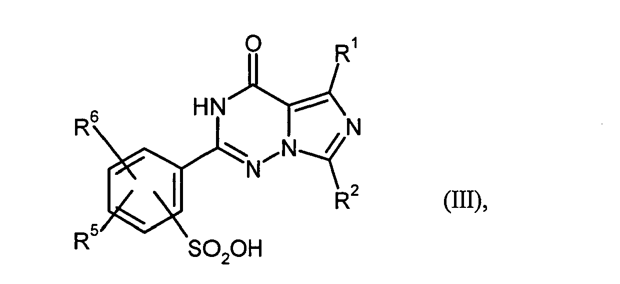 in which
R¹, R², R⁵ and R⁶ have the meanings indicated above,
and then with thionyl chloride and the product thus obtained is reacted in situ in an inert solvent with an amine of the formula (IV) 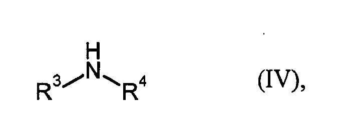 in which
R³ and R⁴ have the meaning indicated above,
and, if appropriate, reacted to give the corresponding salts, hydrates or N-oxides.

2. Process according to Claim 1, **characterized in that**
R¹ denotes hydrogen or straight-chain or branched alkyl having up to 4 carbon atoms,
R² denotes straight-chain alkyl having up to 4 carbon atoms,
R³ and R⁴ identically to or differently from one another denote a straight-chain or branched alkyl chain having up to 5 carbon atoms, which is optionally substituted up to two times in an identical or different manner by hydroxyl or methoxy,
or
R³ and R⁴ , together with the nitrogen atom, form a piperidinyl or morpholinyl ring or a radical of the formula 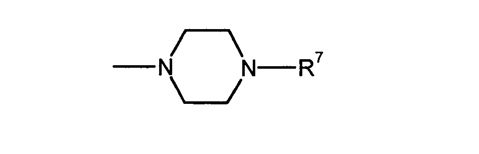 in which
R⁷ denotes hydrogen, straight-chain or branched alkyl having up to 4 carbon atoms, which is optionally mono- or disubstituted, in an identical or different manner, by hydroxyl, straight-chain or branched alkoxy each having up to 4 carbon atoms, or denotes C₃₋₆-cycloalkyl,
and the heterocycles mentioned under R³ and R⁴, formed together with the nitrogen atom, are optionally mono- or disubstituted, in an identical or different manner, if appropriate also geminally, by hydroxyl, straight-chain or branched acyl or alkoxycarbonyl each having up to 4 carbon atoms, optionally by straight-chain or branched alkyl having up to 4 carbon atoms, which is optionally mono- or disubstituted, in an identical or different manner, by hydroxyl,
R⁵ and R⁶ identically to or differently from one another denote hydrogen, straight-chain or branched alkyl having up to 6 carbon atoms, hydroxyl or straight-chain or branched alkoxy having up to 6 carbon atoms.

3. Process according to Claim 1, **characterized in that**
R¹ denotes hydrogen or straight-chain or branched alkyl having up to 4 carbon atoms,
R² denotes straight-chain alkyl having up to 4 carbon atoms,
R³ and R⁴ identically to or differently from one another denote methyl or ethyl, which are optionally substituted up to two times in an identical or different manner by hydroxyl,
or
R³ and R⁴, together with the nitrogen atom, form a piperidinyl or morpholinyl ring or a radical of the formula 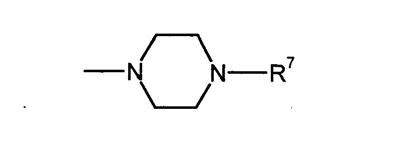 in which
R⁷ denotes hydrogen, methyl or ethyl, which is optionally mono-or disubstituted, in an identical or different manner, by hydroxyl, methoxy or ethoxy, or denotes cyclopentyl or cyclohexyl,
and the heterocycles mentioned under R³ and R⁴, formed together with the nitrogen atom, are optionally mono- or disubstituted, in an identical or different manner, if appropriate also geminally, by hydroxyl, methyl or ethyl,
R⁵ and R⁶ identically to or differently from one another denote hydrogen, straight-chain or branched alkyl having up to 6 carbon atoms, hydroxyl or straight-chain or branched alkoxy having up to 6 carbon atoms.

4. Process according to Claim 1, **characterized in that**
R¹ denotes methyl or ethyl,
R² denotes n-propyl,
R³ and R⁴ identically to or differently from one another denote methyl or ethyl, which are optionally substituted up to two times in an identical or different manner by hydroxyl,
or
R³ and R⁴ , together with the nitrogen atom, form a piperidinyl or morpholinyl ring or a radical of the formula 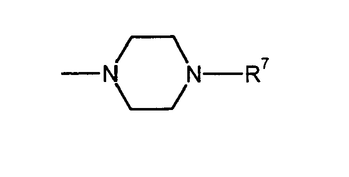 in which
R⁷ denotes hydrogen, methyl or ethyl, which is optionally mono-or disubstituted, in an identical or different manner, by hydroxyl, methoxy or ethoxy, or denotes cyclopentyl or cyclohexyl,
and the heterocycles mentioned under R³ and R⁴, formed together with the nitrogen atom, are optionally mono- or disubstituted, in an identical or different manner, if appropriate also geminally, by hydroxyl, methyl or ethyl,
R⁵ denotes hydrogen,
R⁶ denotes ethoxy.

5. Process according to Claim 1, **characterized in that** the compounds of the formula (II) are carried out by reaction of the compounds of the formula (V) 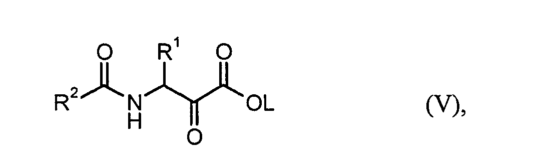 in which
R¹ and R² have the meaning indicated Claim 1
and
L represents straight-chain or branched alkyl having up to 4 carbon atoms,
with compounds of the general formula (VI) 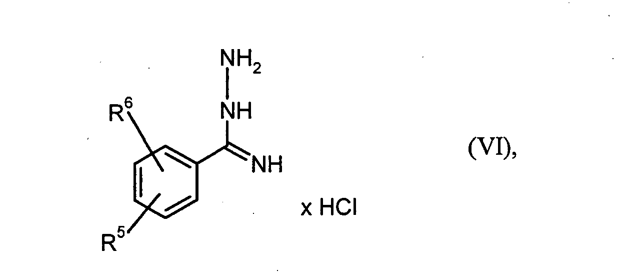 in which
R⁵ and R⁶ have the meaning indicated in Claim 1
in a two-stage reaction in the systems methanol and phosphorous oxychloride/ acetic acid or methanol and acetyl chloride / acetic acid.

## Revendications

1. Procédé de production de composés de formule (I) 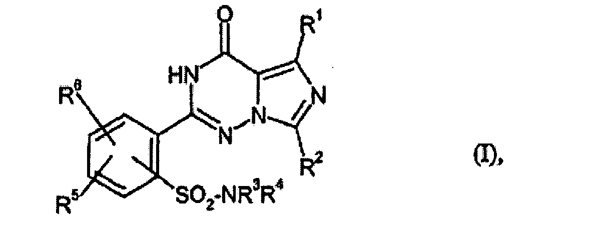 dans laquelle
R¹ représente l'hydrogène ou un reste alkyle linéaire ou ramifié ayant jusqu'à 4 atomes de carbone,
R² représente un reste alkyle linéaire ayant jusqu'à 4 atomes de carbone,
R³ et R⁴ sont identiques ou différents et représentent une chaîne alkylique linéaire ou ramifiée ayant jusqu'à 5 atomes de carbone, qui est éventuellement substituée jusqu'à deux fois identiques ou différentes par un groupe hydroxy ou méthoxy,
ou bien
R³ et R⁴ forment, conjointement avec l'atome d'azote, un noyau pipéridinyle, morpholinyle, thiomorpholinyle ou un reste de formule 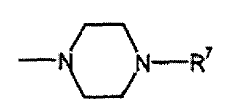 dans laquelle
R⁷ représente l'hydrogène, un groupe formyle, un reste acyle ou alkoxycarbonyle linéaire ou ramifié ayant chacun jusqu'à 6 atome de carbone, ou un reste alkyle linéaire ou ramifié ayant jusqu'à 6 atomes de carbone, qui est éventuellement substitué une ou deux fois identiques ou différentes par un groupe hydroxy, carboxyle, alkoxy ou alkoxycarbonyle linéaire ou ramifié ayant chacun jusqu'à 6 atomes de carbone, ou un reste cycloalkyle en C₃ à C₈,
et les hétérocycles indiqués pour R³ et R⁴, formés conjointement avec l'atome d'azote, sont éventuellement substitués une ou deux fois identiques ou différentes, de façon éventuellement géminée, par un groupe hydroxy, formyle, carboxyle, acyle ou alkoxycarbonyle linéaire ou ramifié ayant chacun jusqu'à 6 atomes de carbone,
et/ou les hétérocycles indiqués pour R³ et R⁴, formés conjointement avec l'atome d'azote, sont éventuellement substitués par un reste alkyle linéaire ou ramifié ayant jusqu'à 6 atomes de carbone, qui est éventuellement substitué une ou deux fois identiques ou différentes par un groupe hydroxy ou carboxyle,
et/ou les hétérocycles indiqués pour R³ et R⁴, formés conjointement avec l'atome d'azote, sont éventuellement substitués par un groupe pipéridinyle ou pyrrolidinyle éventuellement lié par l'intermédiaire de l'azote,
R⁵ et R⁶ sont identiques ou différents et représentent l'hydrogène, un reste alkyle linéaire ou ramifié ayant jusqu'à 6 atomes de carbone, un groupe hydroxy ou un groupe alkoxy linéaire ou ramifié ayant jusqu'à 6 atomes de carbone,
**caractérisé en ce que** des composés de formule (II) 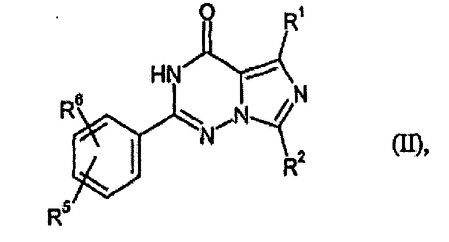 dans laquelle
R¹, R², R⁵ et R⁶ ont les définitions indiquées ci-dessus, sont amenés à réagir avec de l'acide sulfurique pour former des composés de formule (III) 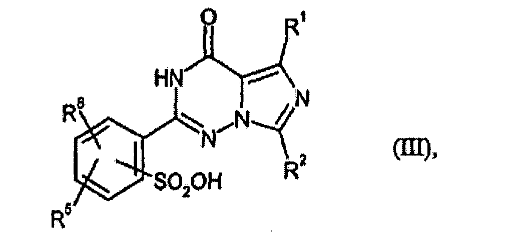 dans laquelle
R¹, R², R⁵ et R⁶ ont les définitions indiquées ci-dessus, puis avec le chlorure de thionyle, et le produit ainsi obtenu est amené à réagir in situ dans un solvant inerte avec une amine de formule (IV) 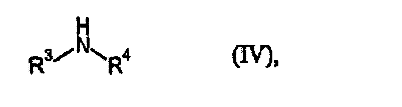 dans laquelle
R³ et R⁴ ont la définition indiquée ci-dessus, ainsi que pour former éventuellement des sels, hydrates ou N-oxydes correspondants.

2. Procédé suivant la revendication 1, **caractérisé en ce que** :
R¹ représente l'hydrogène ou un reste alkyle linéaire ou ramifié ayant jusqu'à 4 atomes de carbone,
R² représente un reste alkyle linéaire ayant jusqu'à 4 atomes de carbone,
R³ et R⁴ sont identiques ou différents et représentent une chaîne alkylique linéaire ou ramifiée ayant jusqu'à 5 atomes de carbone, qui est éventuellement substituée jusqu'à deux fois identiques ou différentes par un groupe hydroxy ou méthoxy,
ou bien
R³ et R⁴ forment, conjointement avec l'atome d'azote, un noyau pipéridinyle, morpholinyle ou un reste de formule 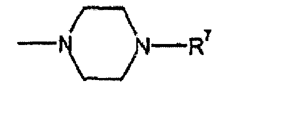 dans laquelle
R⁷ représente l'hydrogène, un reste alkyle linéaire ou ramifié ayant jusqu'à 4 atome de carbone, qui est éventuellement substitué une ou deux fois identiques ou différentes par un groupe hydroxy, un groupe alkoxy linéaire ou ramifié ayant dans chaque cas jusqu'à 4 atomes de carbone, ou un reste cycloalkyle en C₃ à C₆,
et les hétérocycles indiqués pour R³ et R⁴, formés conjointement avec l'atome d'azote, sont éventuellement substitués une ou deux fois identiques ou différentes, éventuellement de façon géminée, par un groupe hydroxy, un groupe acyle ou alkoxycarbonyle linéaire ou ramifié ayant chacun jusqu'à 4 atomes de carbone, le cas échéant par un reste alkyle linéaire ou ramifié ayant jusqu'à 4 atomes de carbone, qui est éventuellement substitué une ou deux fois identiques ou différentes par un groupe hydroxy,
R⁵ et R⁶ sont identiques ou différents et représentent l'hydrogène, un reste alkyle linéaire ou ramifié ayant jusqu'à 6 atomes de carbone, un groupe hydroxy ou un reste alkoxy linéaire ou ramifié ayant jusqu'à 6 atomes de carbone.

3. Procédé suivant la revendication 1, **caractérisé en ce que**
R¹ représente l'hydrogène ou un reste alkyle linéaire ou ramifié ayant jusqu'à 4 atomes de carbone,
R² représente un reste alkyle linéaire ayant jusqu'à 4 atomes de carbone,
R³ et R⁴ sont identiques ou différents et représentent un reste méthyle ou un reste éthyle, qui sont éventuellement substitués jusqu'à deux fois identiques ou différentes par un groupe hydroxy,
ou bien
R³ et R⁴ forment, conjointement avec l'atome d'azote, un noyau pipéridinyle, morpholinyle ou un reste de formule 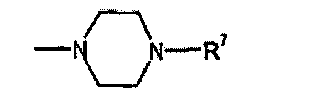 dans laquelle
R⁷ représente l'hydrogène, un reste méthyle ou éthyle, qui est éventuellement substitué une ou deux fois identiques ou différentes par un groupe hydroxy, méthoxy ou éthoxy, ou un reste cyclopentyle ou cyclohexyle,
et les hétérocycles indiqués pour R³ et R⁴, formés conjointement avec l'atome d'azote, sont éventuellement substitués une ou deux fois identiques ou différentes, éventuellement de façon géminée, par un groupe hydroxy, méthyle ou éthyle,
R⁵ et R⁶ sont identiques ou différents et représentent l'hydrogène, un reste alkyle linéaire ou ramifié ayant jusqu'à 6 atomes de carbone, un groupe hydroxy ou un reste alkoxy linéaire ou ramifié ayant jusqu'à 6 atomes de carbone.

4. Procédé suivant la revendication 1, **caractérisé en ce que**
R¹ est un reste méthyle ou éthyle,
R² est un reste n-propyle,
R³ et R⁴ sont identiques ou différents et représentent un reste méthyle ou un reste éthyle, qui sont éventuellement substitués jusqu'à deux fois identiques ou différentes par un groupe hydroxy,
ou bien
R³ et R⁴ forment, conjointement avec l'atome d'azote, un noyau pipéridinyle, morpholinyle ou un reste de formule 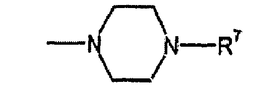 dans laquelle
R⁷ représente l'hydrogène, un reste méthyle ou éthyle, qui est éventuellement substitué une ou deux fois identiques ou différentes par un groupe hydroxy, méthoxy ou éthoxy, ou un reste cyclopentyle ou cyclohexyle,
et les hétérocycles indiqués pour R³ et R⁴, formés conjointement avec l'atome d'azote, sont éventuellement substitués une ou deux fois identiques ou différentes, éventuellement de façon géminée, par un groupe hydroxy, méthyle ou éthyle,
R⁵ représente l'hydrogène
R⁶ représente le reste éthoxy.

5. Procédé suivant la revendication 1, **caractérisé en ce que** les composé de formule (II) sont obtenus par réaction des composés de formule (V) 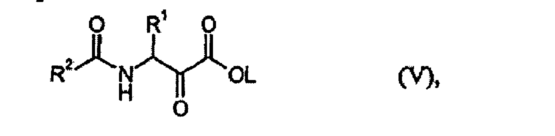 dans laquelle
R¹ et R² ont la définition indiquée dans la revendication 1,
L est un reste alkyle linéaire ou ramifié ayant jusqu'à 4 atomes de carbone,
avec des composés de formule générale (VI) 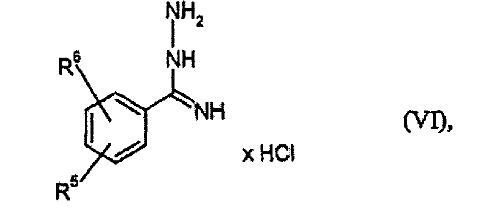 dans laquelle
R⁵ et R⁶ ont la définition indiquée dans la revendication 1, dans une réaction à deux étapes dans les systèmes méthanol et oxytrichlorure de phosphore/acide acétique ou méthanol et chlorure d'acétyle/acide acétique.
